# EUROPEAN PATENT SPECIFICATION

(11) **EP 3 527 231 B1**
(45) Date of publication and mention of the grant of the patent: **25.05.2022**
(21) Application number: 19157343.5
(22) Date of filing: 15.02.2019
(51) Int. Cl.: A61L 2/08, B65B 55/00, B65B 55/08, B65B 9/20

(54) **STERILIZATION APPARATUS, PACKAGING MACHINE HAVING A STERILIZATION APPARATUS FOR PRODUCING SEALED PACKAGES AND METHOD FOR STERILIZING**
STERILISATIONSVORRICHTUNG, VERPACKUNGSMASCHINE MIT EINER STERILISATIONSVORRICHTUNG ZUR HERSTELLUNG VERSIEGELTER VERPACKUNGEN UND VERFAHREN ZUR STERILISATION
APPAREIL DE STÉRILISATION, MACHINE D'EMBALLAGE COMPORTANT UN APPAREIL DE STÉRILISATION POUR PRODUIRE DES EMBALLAGES HERMÉTIQUES ET PROCÉDÉ DE STÉRILISATION

(30) Priority: 19.02.2018 EP 18157357
(43) Date of publication of application: 21.08.2019
(73) Proprietor: Tetra Laval Holdings & Finance S.A., 1009 Pully (CH)
(72) Inventor: RICCO', Marco, 41057 Spilamberto (IT); BULGARELLI, Matteo, 42018 San Martino in Rio (IT)
(74) Representative: Tetra Pak - Patent Attorneys SE

(56) References cited:
- EP-A1- 1 232 760
- EP-A1- 2 889 044
- EP-B1- 1 638 845
- WO-A1-2007/050007
- US-A- 3 757 164

## Description

### TECHNICAL FIELD

The present invention relates to a sterilization apparatus for a packaging machine for producing sealed packages of a pourable product, in particular a pourable food product.

The present invention also relates to a packaging machine for producing sealed packages of a pourable product, in particular a pourable food product, having a sterilization apparatus.

The present invention also relates to a method for sterilizing a web of packaging material from which sealed packages of a pourable product, in particular a pourable food product, are formed.

### BACKGROUND ART

As is known, many liquid or pourable food products, such as fruit juice, UHT (ultra-high-temperature treated) milk, wine, tomato sauce, etc., are sold in packages made of sterilized packaging material.

A typical example is the parallelepiped-shaped package for liquid or pourable food products known as Tetra Brik Aseptic (registered trademark), which is made by sealing and folding laminated strip packaging material. The packaging material has a multilayer structure comprising a base layer, e.g. of paper, covered on both sides with layers of heat-seal plastic material, e.g. polyethylene. In the case of aseptic packages for long-storage products, such as UHT milk, the packaging material also comprises a layer of oxygen-barrier material (an oxygen-barrier layer), e.g. an aluminum foil, which is superimposed on a layer of heat-seal plastic material, and is in turn covered with another layer of heat-seal plastic material forming the inner face of the package eventually contacting the food product.

Packages of this sort are normally produced on fully automatic packaging machines, which advance a web of packaging material from a magazine unit through a sterilization apparatus for sterilizing the web of packaging material and to an isolation chamber (a closed and sterile environment) in which the sterilized web of packaging material is maintained and advanced. During advancement of the web of packaging material through the isolation chamber, the web of packaging material is folded and sealed longitudinally to form a tube having a longitudinal seam portion, which is further fed along a vertical advancing direction.

In order to complete the forming operations, the tube is filled with a sterilized or sterile-processed pourable product, in particular a pourable food product, and is transversally sealed and subsequently cut along equally spaced transversal cross sections within a package forming unit of the packaging machine during advancement along the vertical advancing direction.

Pillow packages are so obtained within the packaging machine, each pillow package having a longitudinal sealing band, a top transversal sealing band and a bottom transversal sealing band.

In the recent years, sterilization apparatus have become available, which are configured to sterilize the web of packaging material by means of the application of physical irradiation, in particular electromagnetic irradiation, even more particular electron beam irradiation.

A typical sterilization apparatus of this kind comprises an irradiation device, having a pair of electron beam emitters spaced apart from one another and defining in cooperation a treatment space through which, in use, the web of packaging material advances. Each one of the electron beam emitters is adapted to direct the respective electron beam onto one respective face of the web of packaging material advancing through the treatment space.

A drawback of such a sterilization apparatus is that in the case that the web of packaging material comprises a conductive layer, typically an aluminum foil acting as an oxygen-barrier layer, the application of the electron beams can lead to the formation of undesired charges on the conductive layer (which may also diffuse onto the outer layers of the web of packaging material), e.g. the formation of the charges can lead to disturbing sensors of the sterilization apparatus, which are configured to detect and/or determine the sterilization efficacy of the irradiation device.

EP2889044 discloses an electron beam sterilization unit for processing food packaging material, the unit having a frame; at least one electron beam emitter fitted to the frame, along the path of the material for processing, and having a flange for connection to the frame; and a locking device, which is fitted to the frame, and has thrust devices for exerting a lock force on the flange in a given first direction, and for locking the emitter, with respect to the frame, in a given work position, and actuating devices for activating the thrust devices and which are defined by toggle devices.

### DISCLOSURE OF INVENTION

It is therefore an object of the present invention to provide a sterilization apparatus for a packaging machine to overcome, in a straightforward and low-cost manner, at least one of the aforementioned drawbacks.

In particular, it is an object of the present invention to provide a sterilization apparatus for a packaging machine for at least reducing the possibility of the formation of electrical charges within and/or on the web of packaging material.

It is a further object of the present invention to provide a packaging machine having a sterilization apparatus to overcome, in a straightforward and low-cost manner, at least one of the aforementioned drawbacks.

It is an even further object of the present invention to provide a method for sterilizing a web of packaging material to overcome, in a straightforward and low-cost manner, at least one of the aforementioned drawbacks.

According to the present invention, there is provided a sterilization apparatus according to claim 1.

According to the present invention, there is also provided a packaging machine according to claim 9.

According to the present invention, there is also provided a method for sterilizing a web of packaging material according to claim 12.

Preferred embodiments are claimed in the dependent claims.

### BRIEF DESCRIPTION OF THE DRAWINGS

A non-limiting embodiment of the present invention will be described by way of example with reference to the accompanying drawings, in which:
Figure 1 is a schematic view of a packaging machine for packaging a pourable product, with parts removed for clarity;
Figure 2 is a first perspective view of a detail of the packaging machine of Figure 1, with parts removed for clarity;
Figure 3 is a second perspective view of the detail of Figure 2, with parts removed for clarity;
Figure 4 is a partially exploded perspective view of the detail of Figures 2 and 3, with parts removed for clarity; and
Figure 5 is a sectionized view of the detail of Figures 2 to 4 during operation, with parts removed for clarity.

### BEST MODES FOR CARRYING OUT THE INVENTION

Number 1 indicates as a whole a packaging machine for producing sealed packages 2 of a pourable product, in particular a pourable food product such as pasteurized milk, fruit juice, wine, tomato sauce, etc., from a tube 3 of a web 4 of packaging material. In particular, in use, tube 3 extends along a longitudinal axis, in particular having a vertical orientation.

Web 4 of packaging material has a multilayer structure (not shown) and comprises at least one conductive layer, in particular one conductive gas- and light-barrier layer, even more particular an aluminum layer.

Preferentially, web 4 further comprises:
- at least one layer of fibrous material, preferably paper;
- at least a first layer of heat-seal plastic material, e.g. polyethylene, superimposed onto one face of the layer of fibrous material and, in particular defining the outer face of package 2 once the package 2 has been formed; and
- at least a second layer of heat-seal plastic material, e.g. polyethylene, superimposed on one face of the conductive layer and, in particular forming the inner face of package 2 eventually contacting the filled food product once formation of package 2 has been terminated.

In particular, the conductive layer is interposed between the layer of fibrous material and the second layer of heat-seal plastic material.

In further detail, web 4 comprises a lateral edge 5 and a lateral edge 6 opposite to lateral edge 5. In particular, lateral edge 5 and lateral edge 6 are transversally separated from one another according to the transversal width of web 4.

In even further detail, lateral edge 5 and lateral edge 6 expose the respective edge portion of at least the conductive layer, in particular also of the layer of fibrous material and the first layer of heat-seal plastic material and the second layer of heat-seal plastic material.

Preferentially, web 4 also comprises a first face 7 and a second face 8, in particular first face 7 being the face of web 4 forming the inner face of the formed package 2 eventually contacting the filled food product.

A typical package 2 obtained by packaging machine 1 comprises a longitudinal seam portion and a pair of transversal sealing bands, in particular a transversal top sealing band and a transversal bottom sealing band.

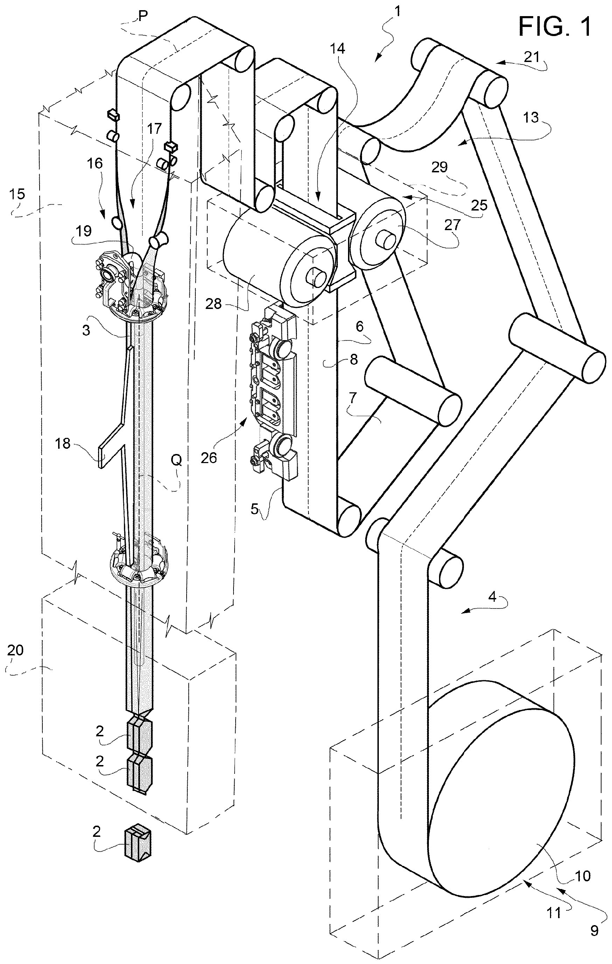

With particular reference to Figure 1, packaging machine 1 is configured to advance web 4 along a web advancement path P, to sterilize web 4 during advancement along path P, to form tube 3 from web 4, to fill tube 3 and to form single packages 2 from the filled tube 3.

Preferentially, packaging machine 1 comprises:
- a magazine unit 9 adapted to host at least one reel 10 of web 4 at a host station 11;
- a sterilization apparatus 13 configured to sterilize web 4 at a sterilization station 14, arranged downstream of host station 11 along path P;
- an isolation chamber 15 separating an inner environment, in particular an inner sterile environment, from an outer environment and being configured to receive the sterilized web 4 from sterilization apparatus 13;

- a tube forming device 16 extending along a longitudinal axis, in particular having a vertical orientation, and being arranged, in particular at a tube forming station 17, at least partially, preferably fully, within isolation chamber 15 and being adapted to form tube 3 from the, in use, advancing and sterilized web 4;
- a sealing device 18 at least partially arranged within isolation chamber 15 and being adapted to longitudinally seal tube 3 formed by tube forming device 16 so as to form a longitudinal seam portion of tube 3;
- filling means 19 for filling tube 3 with the pourable product;
- a package forming unit 20 adapted to at least form and transversally seal tube 3, in particular the, in use, advancing tube 3, for forming packages 2; and
- conveying means 21 for advancing in a known manner web 4 along path P from host station 11 to tube forming station 17, at which, in use, web 4 is formed into tube 3 and to advance tube 3 along a tube advancement path Q towards and at least partially through package forming unit 20.

In particular, isolation chamber 15 is arranged downstream of magazine unit 9 along path P.

Preferentially, sterilization station 14 is arranged upstream of tube forming station 17.

Even more particular, isolation chamber 15 is also arranged downstream of sterilization apparatus 13 (sterilization station 14) along path P.

In particular, package forming unit 20 is arranged downstream of isolation chamber 15 and tube forming device 16 along path Q.

Preferentially, conveying means 21 are adapted to advance tube 3 and any intermediate of tube 3 in a manner known as such along path Q, in particular from tube forming station 17 towards and at least partially through package forming unit 20. In particular, with the wording intermediates of tube 3 any configuration of web 4 is meant prior to obtaining the tube structure and after folding of web 4 by tube forming device 16 has started. In other words, the intermediates of tube 3 are a result of the gradual folding of web 4 so as to obtain tube 3, in particular by overlapping lateral edge 5 and lateral edge 6 of web 4 with one another.

With particular reference to Figure 1, sterilization apparatus 13 comprises:
- an irradiation device 25 arranged in the area of sterilization station 14 and being adapted to sterilize at least first face 7, preferentially also second face 8, by directing an irradiation, in particular electromagnetic irradiation, even more particular electron beam irradiation, onto at least first face 7, preferentially also onto second face 8; and
- a grounding unit 26 adapted to ground, in use, the conductive layer of web 4 and, preferentially being arranged upstream of irradiation device 25 along path P.

In more detail, irradiation device 25 comprises:
- at least a first irradiation emitter, in particular an electron beam emitter 27, configured to direct the irradiation, in particular the electromagnetic irradiation, even more particular the electron beam irradiation, in use, on first face 7; and
- preferentially also a second irradiation emitter, in particular an electron beam emitter 28, configured to direct the irradiation, in particular the electromagnetic irradiation, even more particular the electron beam irradiation, in use, on second face 8.

Electron beam emitter 27 and electron beam emitter 28 are arranged side-by-side and distanced from one another so as to define a treatment space through which, in use, web 4 advances. In particular, electron beam emitter 27 is placed such to face first face 7 and electron beam emitter 28 is placed such to face second face 8. In use, web 4 advances through the treatment space and, accordingly, between electron beam emitter 27 and electron beam emitter 28.

In a preferred embodiment, irradiation device 25 also comprises a sensor device (not shown) adapted to detect and/or determine, in particular inline, the sterilization efficacy of at least electron beam emitter 27, preferentially also of electron beam emitter 28.

More specifically, the sensor device comprises:
- a first sensor associated to and adapted to detect and/or determine the sterilization efficacy of electron beam emitter 27; and
- preferentially also a second sensor associated to and adapted to detect and/or determine the sterilization efficacy of electron beam emitter 28.

Preferably, the first sensor and the second sensor are arranged such that, in use, the first sensor and the second sensor are interposed between the advancing web 4 and respectively the electron beam emitter 27 and the electron beam emitter 28.

Preferentially, electron beam emitter 27 and electron beam emitter 28 are placed within a shielding housing 29 of irradiation device 25 adapted to shield the irradiation emitted by electron beam emitter 27 and electron beam emitter 28.

More specifically, shielding housing 29 comprises an inlet for receiving web 4 and an outlet for allowing exit of web 4.

With particular reference to Figure 1, grounding unit 26 is arranged upstream of irradiation device 25, in particular of electron beam emitter 27 and electron beam emitter 28, along path P.

Preferentially, grounding unit 26 is arranged in the proximity of the inlet of shielding housing 29. Even more preferentially, grounding unit 26 is also arranged outside of shielding housing 29.

In particular, grounding unit 26 is configured to establish contact with at least one of lateral edge 5 and lateral edge 6, in the specific example shown lateral edge 5, along a grounding portion of path P.

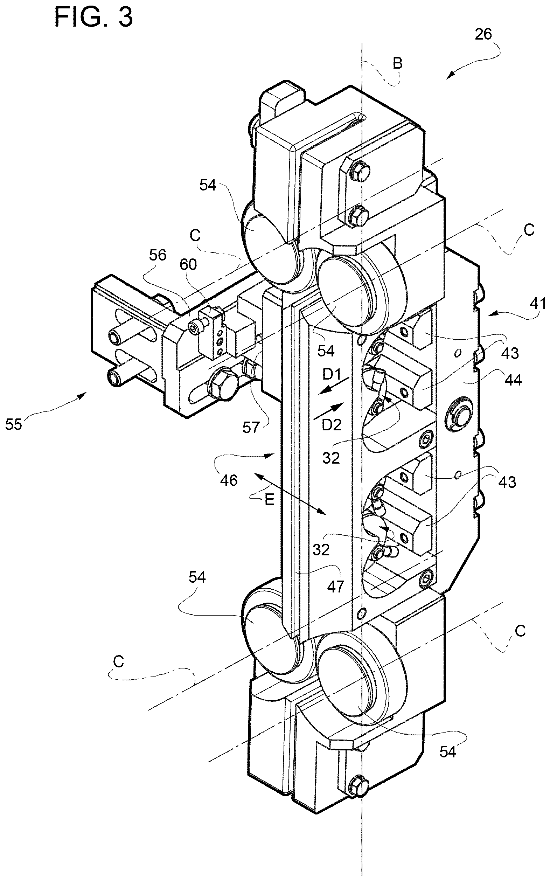

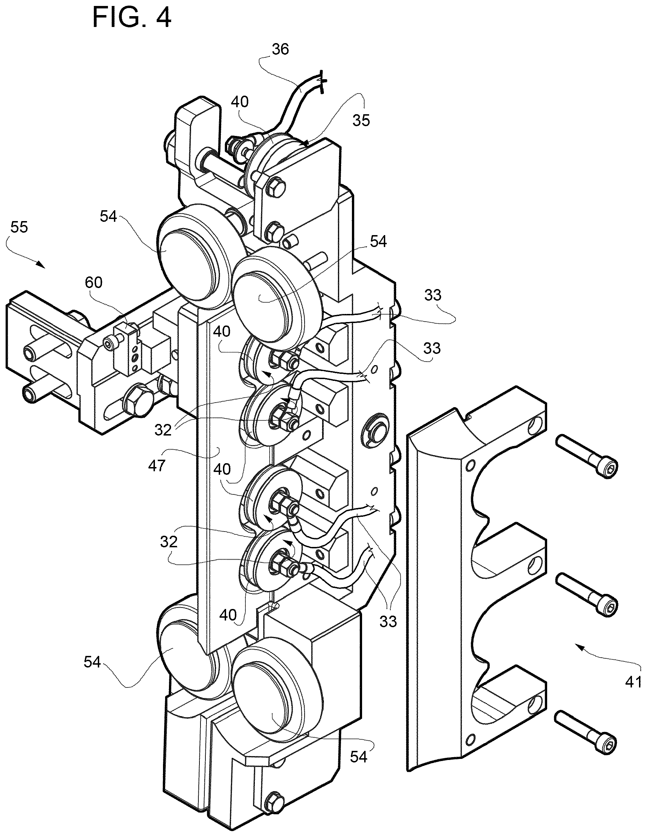

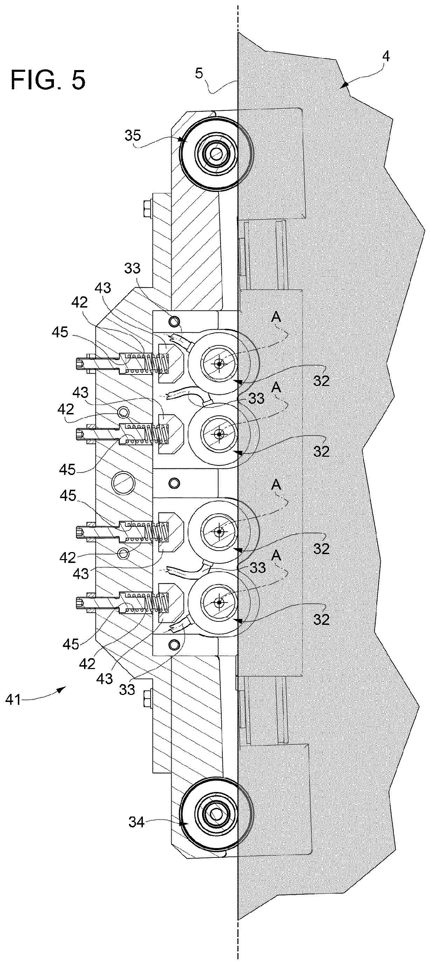

With particular reference to Figures 1 to 5, grounding unit 26 comprises at least one, preferentially a plurality of grounded conductive interaction elements, each one being configured to establish contact with lateral edge 5 so as to electrically connect to the conductive layer for grounding, in use, the conductive layer.

According to the invention, each grounded conductive interaction element is in the form of a grounded conductive wheel 32 being rotatable around a respective rotation axis A, in particular advancing web 4 actuates rotation of conductive wheel 32. Preferentially, each conductive wheel 32 is of a steel material.

In particular, by choosing each interaction element being a conductive wheel 32 and being rotatable around the respective axis A it is possible to substantially avoid any wear of web 4, in particular lateral edge 5.

Preferentially, conductive wheels 32 are arranged in succession of one another (arranged in a row) and configured to contemporaneously contact different zones of lateral edge 5. In other words, in use, conductive wheels 32 are arranged in succession of one another along path P.

Even though also the presence of one conductive wheel 32 allows to obtain satisfying results, by providing for a plurality of conductive wheels 32 arranged in a row an even more efficient grounding of the conductive layer is achieved as the contact is established over a longer extension of lateral edge 5.

More specifically, grounding unit 26 extends along an extension axis B with conductive wheels 32 being arranged in succession along axis B. In particular, in use, axis B is parallel to lateral edge 5, in particular parallel to the part of lateral edge 5 advancing along the grounding portion of path P.

In a preferred embodiment, grounding unit 26 comprises at least one, preferentially a plurality of conductive cables 33, each one connected to one respective conductive wheel 32 and to the ground for grounding the respective conductive wheel 32.

In a preferred embodiment, grounding unit 26 also comprises at least one inlet guide wheel 34 and at least one outlet guide wheel 35 being adapted to at least partially guide advancement of web 4 along path P, in particular along the grounding portion of path P, preferentially by engaging with lateral edge 5.

In more detail, inlet guide wheel 34 and outlet guide wheel 35 are distanced from one another, in particular along axis B. In use, grounding unit 26 is arranged such that inlet guide wheel 33 is arranged upstream of outlet guide wheel 34 along path P.

Preferentially, inlet guide wheel 34 and outlet guide wheel 35 are grounded and conductive and are configured to establish contact with lateral edge 5 and to electrically connect to the conductive layer of web 4 for grounding the conductive layer. Even more particularly, inlet guide wheel 34 and outlet guide wheel 35 are grounded by means of a respective auxiliary conductive cable 36 of grounding unit 26.

Preferentially, conductive wheels 32 are interposed between inlet guide wheel 34 and outlet guide wheel 35 with respect to axis B.

In a preferred embodiment, conductive wheels 32, preferentially also inlet guide wheel 34 and outlet guide wheel 35, comprise a respective central groove 40 configured to receive, in use, lateral edge 5.

With particular reference to Figures 1 to 5, grounding unit 26 also comprises a support structure 41 carrying, in particular moveably carrying, conductive wheels 32; and preferentially also carrying, in particular fixedly carrying, inlet guide wheel 34 and outlet guide wheel 35.

Preferentially, grounding unit 26 also comprises movement means for allowing movement of conductive wheels 32, in a direction D1 and a direction D2 opposite to direction D1. Preferentially, direction D1 and direction D2 are transversal, in particular orthogonal to axis B. In other words, in use, direction D1 and direction D2 are transversal, in particular orthogonal to lateral edge 5.

Preferably, grounding unit 26 comprises a plurality of elastic members, in particular spring elements 42, each one coupled to one respective conductive wheel 32 and support structure 41 and being configured to allow for a movement of the respective conductive wheel 42 along direction D1 or along direction D2. In particular spring element 42 are part of the movement means of grounding unit 26.

In even other words, each spring element 42 is configured to allow for movement of the respective conductive wheel 32 towards and away from lateral edge 5 so as to guarantee that the force exerted by the respective conductive wheel 32 on lateral edge 5 remains within a predefined limit. In this way, it is avoided that conductive wheels 32 contacting lateral edge 5 may damage lateral edge 5.

In a preferred embodiment, spring elements 42 are configured to bias, in use, the respective conductive wheels 32 against lateral edge 5.

In more detail, grounding unit 26 comprises at least one, preferentially a plurality of single carrier elements 43, each one rotatably carrying one respective conductive wheel 32 and each one being connected to one respective spring element 42 so as to couple the respective conductive wheel 32 to the respective spring element 42.

In even further detail, each support structure 41 comprises a base plate 44 indirectly carrying conductive wheels 32.

Preferentially, base plate 44 has a plurality of housing cavities 45, in particular arranged in a row along axis B, and each one being configured to at least partially house one respective spring element 42.

Preferably, direction D1 and direction D2 are transversal, in particular orthogonal to base plate 44.

In use, at least a respective portion of each conductive wheel 32 is interposed between base plate 44 and lateral edge 5.

In a preferred embodiment, grounding unit 26 also comprises a delimiting assembly 46 configured to limit, in use, movement of web 4 along a delimiting direction E normal to first face 7 and second face 8 of web 4, in particular for ensuring contact of lateral edge 5 with conductive wheels 32.

In more detail, delimiting assembly 46 comprises a first delimiting surface 47 and a second delimiting surface 48 arranged side-by-side and parallel to one another and configured to limit in cooperation the movement of web 4 along delimiting direction E.

In even more detail, first delimiting surface 47 and second delimiting surface 48 define a gap 49 through which, in use, a portion of web 4, in particular lateral edge 5 advances.

Preferentially, conductive wheels 32 are arranged in the proximity of delimiting assembly 46.

Even more preferentially, a respective portion of each conductive wheel 32 is interposed between first delimiting surface 47 and second delimiting surface 48. In other words, the respective portion partially extends into gap 49.

Preferably, another respective portion of each conductive wheel 32 is interposed between delimiting assembly 46 and base plate 44.

In particular, in use, first delimiting surface 47 and second delimiting surface 48 are parallel to web 4.

Preferentially, delimiting assembly 46 is mounted to support structure 41, in particular to base plate 44, even more particular delimiting assembly 46 protrudes away from base plate 44.

With particular reference to Figures 1 to 5, grounding unit 26 also comprises a clamping device 53 connected to support structure 41 and being configured to clamp, in use, web 4.

Preferentially, clamping device 53 has at least one pair of clamp wheels 54, each one rotatable around a respective rotation axis C, in particular being transversal, even more particular perpendicular to rotation axes A (i.e. each rotation axis C is parallel to first face 7 and second face 8). The pair of clamp wheels 54 is configured to clamp web 4 between clamp wheels 54 themselves. In use, advancing web 4 actuates rotation of clamp wheels 54 around rotation axis C.

Preferentially, clamping device 53 also comprises at least one biasing element (not shown) associated to the pair of clamping wheels 54 for biasing the respective clamp wheels 54 towards one another so as to exert the clamping force.

Preferentially, each clamp wheel 54 comprises an outer surface made of a polymeric material.

In the specific example shown, grounding unit 26 comprises two pairs of clamp wheels 54, one being arranged in the proximity of inlet guide wheel 34 and the other one being arranged in the proximity of inlet guide wheel 35. In particular, the two pairs of clamp wheels 54 are interposed between inlet guide wheel 34 and outlet guide wheel 35 with respect to axis B.

Preferentially, conductive wheels 32 are interposed between the two pairs of clamp wheels 54.

Even more preferentially, also delimiting assembly 46 is interposed between the two pairs of clamp wheels 54.

As will be explained in the following, by providing for clamping device 53 it is possible to transfer, in particular in a passive manner, any fluctuation of web 4 in a direction parallel to direction D1 and direction D2, to a movement of at least conductive wheels 32 along a direction D1 or direction D2.

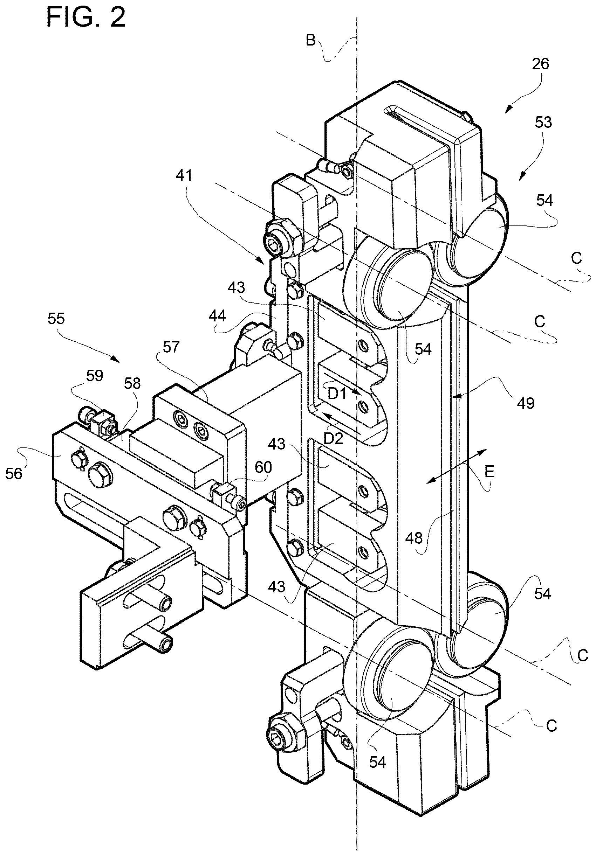

With particular reference to Figures 2 to 4, grounding unit 26, also comprises a mounting assembly 55 for mounting grounding unit 26 to a support device of packaging machine 1, in particular sterilization apparatus 13.

Preferentially, mounting assembly 55 is also connected to, in particular carries, support structure 41.

Even more preferentially, mounting assembly 55 is also connected to the support device.

Preferentially, mounting assembly 55 is also configured to allow for movement of at least conductive wheels 32, in particular of support structure 41, along a mounting direction transversal to path P and to an axis normal to faces 7 and 8, in particular the direction being parallel to D1 and D2.

In more detail, mounting assembly 55 comprises a fixing portion 56 (adapted to be) mounted to the support device and a coupling portion 57 coupled to support structure 41. In particular, coupling portion 57 is moveably coupled to fixing portion 57 and is configured to allow for movement of conductive wheels 32 along the mounting direction.

In even more detail, coupling portion 57 is moveable between a first limit position and a second limit position.

Preferentially, coupling portion 57 has coupling element having a C-like configuration and moveably engaging a guide bar 58 of fixing portion 56, the latter being configured to guide the movement of coupling portion 57 between the first limit position and the second limit position. In particular, guide bar 58 extends parallel to direction D1 and direction D2.

Even more preferentially, fixing portion 56 comprises a first abutment element 59 and a second abutment element 60 configured to define respectively the first limit position and the second limit position. In particular, first abutment element 59 and second abutment element 60 are mounted to guide bar 58 respectively at a first end and a second end of guide bar 58, the second end being opposite to the first end.

As clamping device 53 clamps web 4, any fluctuation of web 4 in a direction parallel to direction D1 or direction D2 is transformed into a movement of coupling portion 57, which again results in movement of at least conductive wheels 32 into direction D1 or direction D2, in particular into a movement of support structure 41 and all the components carried by support structure 41.

In particular, clamping device 53 and mounting assembly 55 define a portion of the movement means of grounding unit 26.

In use, packaging machine 1 forms packages 2 filled with the pourable product.

In more detail, a method for forming packages 2 comprises the following steps:
- advancing web 4 along advancement path P;
- sterilizing web 4 at sterilization station 14;
- forming tube 3 at tube forming station 17;
- longitudinally sealing tube 3;
- filling tube 3 with the pourable product;
- advancing tube 3 along path Q; and
- obtaining single packages 2 from tube 3 by forming tube 3, transversally sealing tube 3 between successive packages 2 and transversally cutting tube 3 between successive packages 2 for obtaining single packages 2.

In more detail, during the step of advancing web 4, conveying means 21 advance web 4 from magazine unit 9 along advancement path P towards sterilization apparatus 13 and tube forming device 16.

In further detail, conveying means 21 advance web 4 from host station 11 to tube forming station 17 through sterilization station 14.

During the step of forming tube 3, tube forming device 16 gradually overlaps lateral edge 5 and lateral edge 6 with one another so as to form a longitudinal seam portion.

During the step of longitudinally sealing tube 3, sealing device 18 thermally seals the longitudinal seam portion.

During the step of advancing tube 3, conveying means 21 advance tube 3 (and the intermediates of tube 3) along path Q to package forming unit 20.

During the step of filling tube 3, filling means 19 fill the pourable product into the longitudinally sealed tube 3.

During the step of obtaining single packages 2, package forming unit 20 forms and transversally seals tube 3 between successive packages 2 and, preferentially, also transversally cuts tube 3 between successive packages 2.

In more detail, during the step of sterilizing web 4, irradiation device 25 directs the irradiation, in particular the electromagnetic irradiation, even more particular the electron beam irradiation, at least onto first face 7, preferentially also onto second face 8 for sterilizing first face 7 and second face 8.

In even more detail, electron beam emitter 27 directs the electron beam irradiation onto first face 7, and preferentially electron beam emitter 28 directs the electron beam irradiation onto second face 8.

Furthermore, during the step of sterilizing web 4, the conductive layer of web 4 is grounded, in particular by grounding unit 26.

More specifically, conductive wheels 32 contact lateral edge 5, establish electrical contact with the conductive layer, and thereby ground the conductive layer of web 4.

While conductive wheels 32 contact lateral edge 5, conductive wheels 32 move along direction D1 or direction D2 so as to follow occurring fluctuations of web 4 along a direction parallel to direction D1 or direction D2.

The movement of conductive wheels 32 along direction D1 or direction D2 is achieved by means of the action of the respective spring elements 42. The movement of conductive wheels 42 along direction D1 or direction D2 is also achieved by clamping device 53 clamping web 4 and mounting assembly 55 allowing movement of support structure 41 as a result of the occurring fluctuation of web 4.

The advantages of sterilization apparatus 13 according to the present invention will be clear from the foregoing description.

In particular, by grounding the conductive layer by means of grounding unit 26, the formation of charges during the sterilization of web 4 by irradiation device 25 is avoided. This leads to an improvement of the signals of the sensor device of irradiation device 25 and, accordingly, an improvement of the inline determination of the sterilization efficacy of irradiation device 25.

Another advantage resides in providing for a plurality of conductive wheels 32 being arranged in a row, so as to increase the extension of the portion of lateral edge 5 being electrically connected to the ground.

A further advantage resides in providing for a respective spring element 42 for each conductive wheel 32 allowing to limit the force exerted by the respective conductive wheel 32 onto lateral edge 5 and to guarantee contact between the respective conductive wheel 32 and lateral edge 5.

An even further advantage is seen in the clamping of web 4 by means of the clamping device 53 and mounting assembly 55 having a coupling portion 57 being moveable with respect to fixing portion 56. In this way, support structure 41 and all the components of grounding unit 26 carried by support structure 41 move in a passive and simultaneous manner so as to follow occurring fluctuations of web 4.

Clearly, changes may be made to packaging machine 1, in particular sterilization apparatus 13 as described herein without, however, departing from the scope of protection as defined in the accompanying claims.

## Claims

1. A sterilization apparatus (13) for sterilizing a web (4) of packaging material having at least one conductive layer and advancing along a web advancement path (P);
the sterilization apparatus (13) comprises at least one irradiation device (25) adapted to direct a sterilization irradiation onto at least one face (7, 8) of the, in use, advancing web (4) of packaging material;
**characterized by** further comprising a grounding unit (26) having at least one grounded and conductive interaction element (32), wherein the interaction element is a grounded conductive wheel (32) rotatable around a rotation axis (A) perpendicular to the at least one face (7,8) of the web (4) of packaging material and configured to establish contact with a lateral edge (5, 6) of the web (4) of packaging material so as to electrically connect to the at least one conductive layer for grounding, in use, the at least one conductive layer.

2. The sterilization apparatus according to claim 1, wherein the grounding unit (26) further comprises a support structure (41) moveably carrying the interaction element (32) and an elastic member (42) coupled to the interaction element (32) and the support structure (41) and configured to determine for a rectilinear movement of the interaction element (42).

3. The sterilization apparatus according to any one of the preceding claims, wherein the grounding unit (26) comprises a plurality of interaction elements (32) arranged in succession of one another and configured to contemporaneously contact different zones of the lateral edge (5, 6).

4. The sterilization apparatus according to any one of the preceding claims, wherein the grounding unit (26) further comprises a delimiting assembly (46) configured to limit movement of the web (4) of packaging material along a delimiting direction (E) normal to the at least one face (7, 8) of the web (4) of packaging material;
the delimiting assembly (46) comprises a first delimiting surface (47) and a second delimiting surface (48) limiting in cooperation the movement of the web (4) of packaging material along the delimiting direction (E); and
wherein the first delimiting surface (47) and the second delimiting surface (48) are arranged side-by-side and distanced from one another so as to define a gap (49) through which, in use, at least the lateral edge (5, 6) of the web (4) of packaging material advances.

5. The sterilization apparatus according to any one of the preceding claims, wherein the grounding unit (26) further comprises at least one grounded and conductive inlet guide wheel (34) and at least one grounded and conductive outlet guide wheel (35) distanced from the inlet guide wheel (34) and each one of the inlet guide wheel (34) and the outlet guide wheel (35) being adapted to at least partially guide advancement of the web (4) of packaging material;
wherein the inlet guide wheel (34) and the outlet guide wheel (35) are also configured to establish contact with the lateral edge (5, 6) of the web (4) of packaging material and to electrically connect to the conductive layer of the web (4) of packaging material; and
wherein the interaction element (32) is interposed between the inlet guide wheel (34) and the outlet guide wheel (35).

6. The sterilization apparatus according to any one of the preceding claims, wherein the grounding unit (26) comprises a support structure (41) carrying the interaction element (32) and a mounting assembly (55) for mounting the grounding unit (26) to a support device;
wherein the mounting assembly (55) comprises a fixing portion (56) adapted to be fixedly mounted to the support device and a coupling portion (57) coupled to a support structure (41) of the grounding unit (26) carrying the interaction element (32);
wherein the coupling portion (57) is moveably coupled to the fixing portion (56) and is configured to actuate movement of the interaction element (32) along a direction (D1, D2) transversal to the web advancement path (P) and to an axis normal to the at least one face (7, 8) of the web (4) of packaging material.

7. The sterilization apparatus according to claim 6, wherein the grounding unit (26) also comprises a clamping device (53) connected to the support structure (41) ;
wherein the clamping device (53) has at least one pair of clamp wheels (54), each one rotatable around a respective rotation axis (C), and being configured to clamp the web (4) of packaging material between the clamp wheels (54) themselves.

8. The sterilization apparatus according to claim 7, wherein the clamping device (53) also comprises at least one biasing element for biasing the clamp wheels (54) towards one another.

9. A packaging machine (1) for producing sealed packages (2) of a pourable product from a web of packaging material having at least one conductive layer and advancing along a web advancement path comprising:
- an isolation chamber (15) separating an inner environment from an outer environment;
- a tube forming device (16) at least partially arranged within the isolation chamber (15) at a tube forming station (17) and being adapted to form a tube (3) from a web (4) of packaging material;
- a sealing device (18) at least partially arranged within the isolation chamber (15) and being adapted to longitudinally seal the tube (3) formed by the tube forming device (16);
- filling means (19) for filling the tube (3) with the pourable product;
- a package forming unit (20) adapted to form and to transversally seal the tube (3) for forming the packages (2);
- conveying means (21) for advancing the web (4) of packaging material along the web advancement path (P) from a magazine unit (9) to the tube forming device (16) and for advancing the tube (3) along a tube advancement path (Q) to the package forming unit (20);
**characterized by** further comprising a sterilization apparatus (13) according to any one of claims 1 to 8.

10. The packaging machine according to claim 9, wherein the grounding unit (26) is arranged upstream of the irradiation device (25) along the web advancement path (P).

11. The packaging machine according to claim 10,
wherein the irradiation device (25) is arranged within a shielding housing (29) having an inlet for receiving the web (4) of packaging material and an outlet for allowing the exit of the web (4) of packaging material;
wherein the grounding unit (26) is arranged in the proximity of the inlet and outside of the shielding housing (29).

12. Method for sterilizing a web (4) of packaging material having at least one conductive layer and advancing along a web advancement path (P), the method comprises the step of:
- directing a sterilizing irradiation onto at least one face (7, 8) of the advancing web (4) of packaging material;
**characterized in that** it further comprised the step of:
- grounding the at least one conductive layer;
wherein the step of grounding the at least one conductive layer is carried out by means of a grounding unit (26) having at least one grounded and conductive interaction element (32), wherein the interaction element is a grounded conductive wheel (32) rotatable around a rotation axis (A) perpendicular to the at least one face (7,8) of the web (4) of packaging material and configured to establish contact with a lateral edge (5, 6) of the web (4) of packaging material so as to electrically connect to the at least one conductive layer for grounding, in use, the at least one conductive layer.

## Patentansprüche

1. Sterilisationsvorrichtung (13) zum Sterilisieren einer Bahn (4) aus Verpackungsmaterial mit mindestens einer leitfähigen Schicht und Vorschieben entlang eines Bahnvorschubpfads (P);
wobei die Sterilisationsvorrichtung (13) mindestens eine Bestrahlungsvorrichtung (25) umfasst, die vorgesehen ist, um eine Sterilisationsbestrahlung auf mindestens eine Seite (7, 8) der bei Gebrauch vorschiebenden Bahn (4) des Verpackungsmaterials zu lenken;
**dadurch gekennzeichnet, dass** sie des Weiteren eine Erdungseinheit (26) mit mindestens einem geerdeten und leitfähigen Interaktionselement (32) umfasst, wobei das Interaktionselement ein geerdetes leitfähiges Rad (32) ist, das um eine Rotationsachse (A) senkrecht zu der mindestens einen Seite (7, 8) der Bahn (4) des Verpackungsmaterials rotierbar ist und ausgestaltet ist, um Kontakt mit einem lateralen Rand (5, 6) der Bahn (4) des Verpackungsmaterials herzustellen, um so die mindestens eine leitfähige Schicht zur Erdung bei Gebrauch mit der mindestens einen leitfähigen Schicht elektrisch zu verbinden.

2. Sterilisationsvorrichtung nach Anspruch 1, wobei die Erdungseinheit (26) des Weiteren eine Trägerstruktur (41) umfasst, welche beweglich das Interaktionselement (32) und ein elastisches Element (42) trägt, das an das Interaktionselement (32) und die Trägerstruktur (41) gekoppelt ist und ausgestaltet ist, um eine geradlinie Bewegung des Interaktionselements (42) zu bestimmen.

3. Sterilisationsvorrichtung nach einem der vorhergehenden Ansprüche, wobei die Erdungseinheit (26) eine Vielzahl von Interaktionselementen (32) umfasst, die aufeinander folgend angeordnet sind und ausgestaltet sind, um zeitgleich unterschiedliche Zonen des lateralen Randes (5, 6) zu kontaktieren.

4. Sterilisationsvorrichtung nach einem der vorhergehenden Ansprüche, wobei die Erdungseinheit (26) des Weiteren eine Begrenzungsanordnung (46) umfasst, die ausgestaltet ist, um die Bewegung der Bahn (4) des Verpackungsmaterials entlang einer Begrenzungsrichtung (E), die normal zu der mindestens einen Seite (7, 8) der Bahn (4) des Verpackungsmaterials ist, zu begrenzen;
wobei die Begrenzungsanordnung (46) eine erste Begrenzungsoberfläche (47) und eine zweite Begrenzungsoberfläche (48) umfasst, welche zusammenwirkend die Bewegung der Bahn (4) des Verpackungsmaterials entlang der Begrenzungsrichtung (E) begrenzen; und
wobei die erste Begrenzungsoberfläche (47) und die zweite Begrenzungsoberfläche (48) nebeneinander und voneinander beabstandet angeordnet sind, um so einen Spalt (49) zu definieren, durch den bei Gebrauch mindestens der laterale Rand (5, 6) der Bahn (4) des Verpackungsmaterials vorgeschoben wird.

5. Sterilisationsvorrichtung nach einem der vorhergehenden Ansprüche, wobei die Erdungseinheit (26) des Weiteren mindestens ein geerdetes und leitfähiges Einlassführungsrad (34) und mindestens ein geerdetes und leitfähiges Auslassführungsrad (35) umfasst, das von dem Einlassführungsrad (34) beabstandet ist, und wobei jedes von dem Einlassführungsrad (34) und dem Auslassführungsrad (35) eingerichtet ist, um mindestens teilweise Vorschub der Bahn (4) des Verpackungsmaterials zu führen; wobei das Einlassführungsrad (34) und das Auslassführungsrad (35) auch ausgestaltet sind, um Kontakt mit dem lateralen Rand (5, 6) der Bahn (4) des Verpackungsmaterials herzustellen und elektrisch mit der leitfähigen Schicht der Bahn (4) des Verpackungsmaterials verbunden zu werden; und wobei das Interaktionselement (32) zwischen dem Einlassführungsrad (34) und dem Auslassführungsrad (35) angeordnet ist.

6. Sterilisationsvorrichtung nach einem der vorhergehenden Ansprüche, wobei die Erdungseinheit (26) eine Trägerstruktur (41), die das Interaktionselement (32) trägt, und eine Montageanordnung (55) zum Montieren der Erdungseinheit (26) an eine Trägervorrichtung umfasst;
wobei die Montageanordnung (55) einen Fixierabschnitt (56), der eingerichtet ist, um fixiert an der Trägervorrichtung montiert zu werden, und einen Kopplungsabschnitt (57) umfasst, der an eine Trägerstruktur (41) der Erdungseinheit (26) gekoppelt wird, welche das Interaktionselement (32) trägt;
wobei der Kopplungsabschnitt (57) beweglich an den Fixierabschnitt (56) gekoppelt ist und ausgestaltet ist, um Bewegung des Interaktionselements (32) entlang einer Richtung (D1, D2) quer zu dem Bahnvorschubpfad (P) und zu einer Achse, die normal zu der mindestens einen Seite (7, 8) der Bahn (4) des Verpackungsmaterials ist, auszulösen.

7. Sterilisationsvorrichtung nach Anspruch 6, wobei die Erdungseinheit (26) auch eine Klemmvorrichtung (53) umfasst, die mit der Trägerstruktur (41) verbunden ist;
wobei die Klemmvorrichtung (53) mindestens ein Paar Klemmräder (54) aufweist, von denen jedes um eine jeweilige Rotationsachse (C) rotierbar ist und ausgestaltet ist, um die Bahn (4) des Verpackungsmaterials zwischen den Klemmrädern (54) selbst festzuklemmen.

8. Sterilisationsvorrichtung nach Anspruch 7, wobei die Klemmvorrichtung (53) auch mindestens ein Vorspannelement umfasst, um die Klemmräder (54) aufeinander zu vorzuspannen.

9. Verpackungsmaschine (1) zum Produzieren versiegelter Verpackungen (2) eines gießbaren Produkts aus einer Bahn des Verpackungsmaterials mit mindestens einer leitfähigen Schicht und zum Vorschieben entlang eines Bahnvorschubpfads, umfassend:
- eine Isolierkammer (15), die eine innere Umgebung von einer äußeren Umgebung trennt;
- eine Schlauchbildungsvorrichtung (16), die mindestens teilweise innerhalb der Isolierkammer (15) an einer Schlauchbildungsstation (17) angeordnet ist und eingerichtet ist, um aus der Bahn (4) des Verpackungsmaterials einen Schlauch (3) zu bilden;
- eine Versiegelungsvorrichtung (18), die mindestens teilweise innerhalb der Isolierkammer (15) angeordnet ist und eingerichtet ist, um den durch die Schlauchbildungsvorrichtung (16) gebildeten Schlauch (3) in Längsrichtung zu versiegeln;
- Füllmittel (19) zum Füllen des Schlauchs (3) mit dem gießbaren Produkt;
- eine Verpackungsbildungseinheit (20), die eingerichtet ist, um den Schlauch (3) zum Bilden der Verpackungen (2) zu bilden und in Querrichtung zu versiegeln;
- Fördermittel (21) zum Vorschieben der Bahn (4) des Verpackungsmaterials entlang des Bahnvorschubpfads (P) von einer Magazineinheit (9) zu der Schlauchbildungsvorrichtung (16) und zum Vorschieben des Schlauchs (3) entlang eines Schlauchvorschubpfads (Q) zu der Verpackungsbildungseinheit (20); **dadurch gekennzeichnet, dass** sie des Weiteren eine Sterilisationsvorrichtung (13) gemäß einem der Ansprüche 1 bis 8 umfasst.

10. Verpackungsmaschine nach Anspruch 9, wobei die Erdungseinheit (26) vorgeordnet zu der Bestrahlungsvorrichtung (25) entlang des Bahnvorschubpfads (P) angeordnet ist.

11. Verpackungsmaschine nach Anspruch 10,
wobei die Bestrahlungsvorrichtung (25) innerhalb eines Abschirmgehäuses (29) angeordnet ist, das einen Einlass zum Annehmen der Bahn (4) des Verpackungsmaterials und einen Auslass aufweist, damit die Bahn (4) des Verpackungsmaterials austreten kann;
wobei die Erdungseinheit (26) in der Nähe des Einlasses und außerhalb des Abschirmgehäuses (29) angeordnet ist.

12. Verfahren zum Sterilisieren einer Bahn (4) des Verpackungsmaterials mit mindestens einer leitfähigen Schicht und Vorschieben entlang eines Bahnvorschubpfads (P), wobei das Verfahren die Schritte umfasst:
- Lenken einer Sterilisationsbestrahlung auf mindestens eine Seite (7, 8) der vorschiebenden Bahn (4) des Verpackungsmaterials;
**dadurch gekennzeichnet, dass** es des Weiteren den Schritt umfasst:
- Erden der mindestens einen leitfähigen Schicht; wobei der Schritt des Erdens der mindestens einen leitfähigen Schicht mittels einer Erdungseinheit (26) mit mindestens einem geerdeten und leitfähigen Interaktionselement (32) ausgeführt wird, wobei das Interaktionselement ein geerdetes leitfähiges Rad (32) ist, das um eine Rotationsachse (A) senkrecht zu der mindestens einen Seite (7, 8) der Bahn (4) des Verpackungsmaterials rotierbar ist und ausgestaltet ist, um Kontakt mit einem lateralen Rand (5, 6) der Bahn (4) des Verpackungsmaterials herzustellen, um so die mindestens eine leitfähige Schicht zur Erdung bei Gebrauch mit der mindestens einen leitfähigen Schicht elektrisch zu verbinden.

## Revendications

1. Appareil de stérilisation (13) pour stériliser une bande (4) de matériau d'emballage comportant au moins une couche conductrice et avançant le long d'un trajet d'avancement (P) de bande ;
l'appareil de stérilisation (13) comprend au moins un dispositif de rayonnement (25) conçu pour diriger un rayonnement de stérilisation sur au moins une face (7, 8), lors de l'utilisation, de la bande (4) de matériau d'emballage en train d'avancer ;
**caractérisé en ce qu'**il comprend en outre une unité de mise à la terre (26) ayant au moins un élément d'interaction mis à la terre et conducteur (32), dans lequel l'élément d'interaction est une roue conductrice mise à la terre (32) pouvant tourner autour d'un axe de rotation (A) perpendiculaire à l'au moins une face (7, 8) de la bande (4) de matériau d'emballage et configurée pour établir un contact avec un bord latéral (5, 6) de la bande (4) de matériau d'emballage de façon à se connecter électriquement à l'au moins une couche conductrice pour la mise à la terre, lors de l'utilisation, de l'au moins une couche conductrice.

2. Appareil de stérilisation selon la revendication 1, dans lequel l'unité de mise à la terre (26) comprend en outre une structure de support (41) portant de manière mobile l'élément d'interaction (32) et un élément élastique (42) relié à l'élément d'interaction (32) et à la structure de support (41) et configuré pour déterminer un mouvement rectiligne de l'élément d'interaction (42).

3. Appareil de stérilisation selon l'une quelconque des revendications précédentes, dans lequel l'unité de mise à la terre (26) comprend une pluralité d'éléments d'interaction (32) disposés à la suite les uns des autres et configurés pour entrer en contact simultanément avec différentes zones du bord latéral (5, 6).

4. Appareil de stérilisation selon l'une quelconque des revendications précédentes, dans lequel l'unité de mise à la terre (26) comprend en outre un ensemble de délimitation (46) configuré pour limiter le mouvement de la bande (4) de matériau d'emballage le long d'une direction de délimitation (E) normale à l'au moins une face (7, 8) de la bande (4) de matériau d'emballage ;
l'ensemble de délimitation (46) comprend une première surface de délimitation (47) et une seconde surface de délimitation (48) limitant en coopération le mouvement de la bande (4) de matériau d'emballage le long de la direction de délimitation (E) ; et
dans lequel la première surface de délimitation (47) et la seconde surface de délimitation (48) sont disposées côte à côte et espacées l'une de l'autre de sorte à définir un espace (49) à travers lequel avance, lors de l'utilisation, au moins le bord latéral (5, 6) de la bande (4) de matériau d'emballage.

5. Appareil de stérilisation selon l'une quelconque des revendications précédentes, dans lequel l'unité de mise à la terre (26) comprend en outre au moins une roue de guidage d'entrée mise à la terre et conductrice (34) et au moins une roue de guidage de sortie mise à la terre et conductrice (35) éloignée de la roue de guidage d'entrée (34) et chacune de la roue de guidage d'entrée (34) et de la roue de guidage de sortie (35) étant conçue pour guider au moins partiellement l'avancement de la bande (4) de matériau d'emballage ;
dans lequel la roue de guidage d'entrée (34) et la roue de guidage de sortie (35) sont également configurées pour établir un contact avec le bord latéral (5, 6) de la bande (4) de matériau d'emballage et pour se connecter électriquement à la couche conductrice de la bande (4) de matériau d'emballage ; et
dans lequel l'élément d'interaction (32) est interposé entre la roue de guidage d'entrée (34) et la roue de guidage de sortie (35).

6. Appareil de stérilisation selon l'une quelconque des revendications précédentes, dans lequel l'unité de mise à la terre (26) comprend une structure de support (41) portant l'élément d'interaction (32) et un ensemble de montage (55) pour monter l'unité de mise à la terre (26) sur un dispositif de support ;
dans lequel l'ensemble de montage (55) comprend une partie de fixation (56) conçue pour être montée à demeure sur le dispositif de support et une partie d'accouplement (57) accouplée à une structure de support (41) de l'unité de mise à la terre (26) portant l'élément d'interaction (32) ;
dans lequel la partie d'accouplement (57) est accouplée de manière mobile à la partie de fixation (56) et est configurée pour actionner le mouvement de l'élément d'interaction (32) le long d'une direction (D1, D2) transversale au trajet d'avancement (P) de la bande et à un axe normal à l'au moins une face (7, 8) de la bande (4) de matériau d'emballage.

7. Appareil de stérilisation selon la revendication 6, dans lequel l'unité de mise à la terre (26) comprend également un dispositif de serrage (53) relié à la structure de support (41) ;
dans lequel le dispositif de serrage (53) comporte au moins une paire de roues de serrage (54), chacun pouvant tourner autour d'un axe de rotation (C) respectif, et étant configuré pour serrer la bande (4) de matériau d'emballage entre les roues de serrage (54) elles-mêmes.

8. Appareil de stérilisation selon la revendication 7, dans lequel le dispositif de serrage (53) comprend également au moins un élément de sollicitation pour solliciter les roues de serrage (54) l'une vers l'autre.

9. Machine d'emballage (1) pour produire des emballages scellés (2) d'un produit apte au déversement à partir d'une bande de matériau d'emballage ayant au moins une couche conductrice et avançant le long d'un trajet d'avancement de bande comprenant :
- une chambre d'isolement (15) séparant un environnement interne d'un environnement externe ;
- un dispositif de formation (16) de tube disposé au moins partiellement à l'intérieur de la chambre d'isolement (15) au niveau d'une station de formation (17) de tube et étant conçu pour former un tube (3) à partir d'une bande (4) de matériau d'emballage ;
- un dispositif de scellement (18) au moins partiellement disposé à l'intérieur de la chambre d'isolement (15) et conçu pour sceller longitudinalement le tube (3) formé par le dispositif de formation (16) de tube ;
- des moyens de remplissage (19) pour remplir le tube (3) avec le produit apte au déversement ;
- une unité de formation (20) d'emballages conçue pour former et sceller transversalement le tube (3) pour former les emballages (2) ;
- des moyens de transport (21) pour faire avancer la bande (4) de matériau d'emballage le long du trajet d'avancement (P) de la bande depuis une unité de magasin (9) jusqu'au dispositif de formation (16) de tube et pour faire avancer le tube (3) le long d'un trajet d'avancement (Q) de tube jusqu'à l'unité de formation (20) d'emballages ;
**caractérisée en ce qu'**elle comprend en outre un appareil de stérilisation (13) selon l'une quelconque des revendications 1 à 8.

10. Machine d'emballage selon la revendication 9, dans laquelle l'unité de mise à la terre (26) est disposée en amont du dispositif de rayonnement (25) le long du trajet d'avancement (P) de la bande.

11. Machine d'emballage selon la revendication 10,
dans laquelle le dispositif d'exposition (25) est disposé à l'intérieur d'un boîtier de protection (29) ayant une entrée pour recevoir la bande (4) de matériau d'emballage et une sortie pour permettre la sortie de la bande (4) de matériau d'emballage ;
dans laquelle l'unité de mise à la terre (26) est disposée à proximité de l'entrée du boîtier de protection (29) et à l'extérieur de celui-ci.

12. Procédé de stérilisation d'une bande (4) de matériau d'emballage comportant au moins une couche conductrice et avançant le long d'un trajet d'avancement (P) de la bande, le procédé comprend l'étape suivante :
- diriger un rayonnement stérilisant sur au moins une face (7, 8) de la bande (4) de matériau d'emballage en train d'avancer ;
**caractérisé en ce qu'**il comprend en outre l'étape suivante :
- la mise à la terre de l'au moins une couche conductrice ;
dans lequel l'étape de mise à la terre de l'au moins une couche conductrice est réalisée au moyen d'une unité de mise à la terre (26) ayant au moins un élément d'interaction mis à la terre et conducteur (32), dans lequel l'élément d'interaction est une roue conductrice mise à la terre (32) pouvant tourner autour d'un axe de rotation (A) perpendiculaire à l'au moins une face (7, 8) de la bande (4) de matériau d'emballage et configurée pour établir un contact avec un bord latéral (5, 6) de la bande (4) de matériau d'emballage de façon à se connecter électriquement à l'au moins une couche conductrice pour une mise à la terre, lors de l'utilisation, de l'au moins une couche conductrice.
